# EUROPEAN PATENT APPLICATION

(11) **EP 4 843 981 A1**
(43) Date of publication of application: **25.11.2026**
(21) Application number: 26179027.3
(22) Date of filing: 15.05.2026
(51) Int. Cl.: C07D 257/06

(54) **METHOD FOR PREPARATION OF 5,5´-AZOTETRAZOLATE SALTS**

(30) Priority: 20.05.2025 CZ 20250220
(71) Applicant: Sellier & Bellot Int. a.s., 258 01 Vlasim (CZ)
(72) Inventor: Matyas, Robert, 533 42 Zivanice (CZ); Votypka, Adam, 257 64 Zdislavice (CZ); Labaj, Miroslav, 034 01 Ruzomberok (SK); Jalovy, Zdenek, 530 12 Pardubice (CZ)
(74) Representative: Harber IP s.r.o.

(57) **Abstract**

The present invention relates to a method of preparation of 5,5'-azotetrazole salts by oxidation of 5-aminotetrazole using derivatives of isocyanuric acid and halogenated derivatives of *N-*succinimide. 5-Aminotetrazole reacts in an aqueous solution of alkali metal hydroxide with a sodium salt of dichloroisocyanuric acid (SDIC) or with trichloroisocyanuric acid (TCCA) or with N-chlorosuccinimide or with N-bromosuccinimide. The reaction produces the corresponding salt of 5,5'-azotetrazole, wherein the cation is an alkali metal. Said method may be used to prepare also other metal salts, ammonium salt and salts of organic bases of 5,5'-azotetrazole, which are prepared *in situ* from the alkali metal salt of 5,5'-azotetrazole.

## Description

### Field of Art

The present invention relates to a method for preparation of 5,5'-azotetrazolate salts using oxidation of 5-aminotetrazole with derivatives of isocyanuric acid or using halogen derivatives of succinimide. 5,5'-Azotetrazolate salts are used for preparation of pharmaceuticals and explosives, especially as gas-generating rescue systems, components of propellants and initiators or intermediates of these applications. The disclosed method simplifies and reduces the cost of 5,5'-azotetrazolate salts production, as well as of the production of compounds derived from them.

### State of the Art

Salts of 5,5'-azotetrazole are important compounds or intermediates used as components of gas-generating rescue systems, propellants and initiators or intermediates of these applications. Sodium or potassium salt of 5,5'-azotetrazole play the key role, as other salts of 5,5'-azotetrazole are prepared from them by conversion. The sodium salt of 5,5'-azotetrazole is typically prepared by oxidation of 5-aminotetrazole with potassium permanganate, bromine or electrolytically. Sodium and potassium salts of 5,5'-azotetrazole were first prepared by Johannes Thiele in 1898 by oxidation of 5-aminotetrazole with potassium permanganate in an alkaline hydroxide environment at elevated temperature (Thiele J.: Justus Liebigs Ann Chem. 1898, 303, 57-75). Other methods and variations of the procedure are described in the work of Labaj M., Jalový Z., Matyáš R., Nesveda J., Mikuláštík J., Votýpka A.: Org. Process Res. Dev., 2024, 28, 4091-4098).

The yields of reactions from the above sources range from 51-80%. The main disadvantage of this method is the formation of solid manganese dioxide as a by-product of the reaction in a muddy form, which is difficult to filter and difficult to wash. In addition, its filtration from the reaction mixture can only be carried out at high temperatures and requires the use of a relatively large amount of water to wash the product from manganese dioxide, which must be subsequently evaporated or the product precipitated from the solution with large amounts of ethanol to increase the yield. The subsequent recycling and processing of manganese dioxide contaminated with explosive substances is also problematic.

Another published oxidizing agent for the oxidation of 5-aminotetrazole is bromine. This method uses either bromine water, but the product is formed in low yield, or pure bromine, providing a yield of 83% (Wang Y. H., Du Z. M., He Ch. L., Cong X. M., Wang H. S., Wang Ch. Y.: Chin. J. Energ. Mater., 2008, 16, 581-584; Zhao Z., Du Z., Han Z., Zhang Y, He Ch.: J. Energ. Mater, 2016, 34, 183-196). The disadvantage of using bromine is the high toxicity of this substance, even in the form of vapor. In addition, bromine causes severe skin burns (deep ulcers). From an economic point of view, bromine is a relatively expensive oxidizing agent compared to the present invention.

The last option mentioned in the literature is the preparation of sodium 5,5´-azotetrazolate using electrochemical methods. Patent CN107841758 describes the electrolysis of 5-aminotetrazole in sodium hydroxide solution on electrodes made of stable metals such as gold or platinum using a DC or pulsed source with a yield of up to 81 %. The second published method in patent CN108084107 is based on electrolysis combined with photocatalysis in an alkaline hydroxide and carbonate environment with a published yield of 77 %. The disadvantage of the above procedures is the high investment costs for production equipment using electrolysis. Another disadvantage of electrolytic methods is the development of dangerous and explosive hydrogen as a by-product of electrolysis.

The preparation of other salts of 5,5´-azotetrazole, both inorganic and organic, is based on cation exchange from sodium 5,5´-azotetrazolate with chloride, sulfate, or nitrate of the corresponding desired cation. For highly soluble azotetrazolates, a cation exchange reaction using barium 5,5'-azotetrazolate with the corresponding sulfates can also be used, resulting in insoluble barium sulfate and a solution of the corresponding azotetrazolate. The disadvantage of using barium compounds is their toxicity.

The aim of the present invention is to eliminate or at least minimize the above-described disadvantages of the prior art.

### Disclosure of Invention

The present invention relates to a method of preparation of 5,5´-azotetrazole salts by oxidation of 5-aminotetrazole using an oxidizing agent based on chlorinated derivatives of cyanuric acid and halogenated derivatives of *N*-succinimide, in particular using salts of dichloroisocyanuric acid, trichloroisocyanuric acid, *N*-chlorosuccinimide or *N*-bromosuccinimide.

Trichloroisocyanuric acid (TCCA, systematic name 1,3,5-trichloro-1,3,5-triazinane-2,4,6-trione) is commonly used as a disinfection and an algicide, especially in swimming pools and when working with dyes, and also for bleaching in the textile industry.

Dichloroisocyanuric acid (DCCA, systematic name 1,3-dichloro-1,3,5-triazinan-2,4,6-trione) is an oxidizing agent.

Sodium dichloroisocyanurate (SDIC, systematic name sodium-3,5-dichloro-2,4,6-trioxo-1,3,5-triazinan-1-ide) is a white solid used as a stable source of chlorine in disinfectants, biocides, industrial deodorants, and cleaning agents. It is used in small amounts in water disinfection products.

N-Chlorosuccinimide (NCS, systematic name 1-chloropyrrolidine-2,5-dione) is a white solid and is commonly used as a chlorinating and oxidizing agent.

N-Bromosuccinimide (NBC, systematic name 1-bromopyrrolidine-2,5-dione) is used as a brominating agent in organic chemistry.

All of the above compounds are commercially available.

The disclosed method of preparation comprises the following steps: First, 5-aminotetrazole and alkali metal hydroxide (MOH) are dissolved in water with stirring. MOH is preferably selected from the group comprising NaOH, KOH, CsOH, LiOH, RbOH. After cooling the reaction mixture to a temperature in the range of from -10 to 80 °C, the oxidizing agent is gradually added (OxCl or OxBr), the oxidizing agent being selected from the group comprising alkali metal (in particular sodium) salt of dichloroisocyanuric acid, dichloroisocyanuric acid, trichloroisocyanuric acid, *N-*chlorosuccinimide and *N*-bromosuccinimide. Subsequently, the reaction mixture is stirred at a temperature in the range of from -10 to 80 °C, preferably at a temperature of from 0 to 30 °C, more preferably from 0 °C to 10 °C, for at least 5 minutes, preferably for a period of time in the range of from 10 to 90 minutes to form an alkali salt of 5,5'-azotetrazole according to the equation (1). 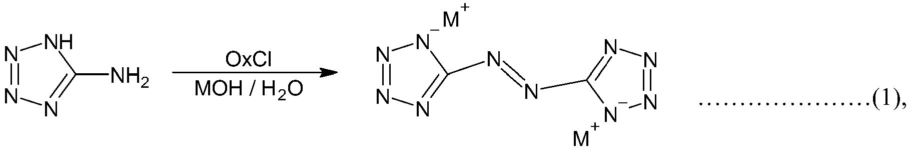 wherein OxCl is an oxidizing agent and MOH is an alkali metal hydroxide.

Preferably, the concentration of the alkali metal hydroxide in the reaction mixture is in the range of from 0.1 to 12 mol/dm³, more preferably the concentration of the alkali metal hydroxide in the reaction mixture is in the range of from 1 to 10 mol/dm³, even more preferably in the range of from 3 to 8 mol/dm³.

Preferably, the initial concentration of 5-aminotetrazole in the reaction mixture is in the range of from 0.1 to 3 mol/dm³, more preferably in the range of from 0.4 to 2 mol/dm³, even more preferably in the range of from 0.5 to 1 mol/dm³, most preferably from 0,6 to 0,8 mol/dm³.

In one embodiment, in particular if SDIC or TCCA is used as the oxidizing agent for the reaction, the final reaction mixture comprising alkaline metal salt of 5,5´-azotetrazole is heated to the temperature in the range of from 30 to 95 °C, preferably to the temperature in the range of from 80 to 90 °C, at which by-products precipitate from the reaction mixture as solids. The solid reaction by-products are then removed from the reaction mixture by filtration. The filtrate containing alkaline metal salt of 5,5´-azotetrazole is then cooled down to the temperature in the range of from 5 to 10 °C, at which said alkaline metal salt of 5,5´-azotetrazole precipitates as a solid and is isolated by filtration.

In one embodiment, when NCS or NBS is used as an oxidizing agent in the reaction, the reaction by-product remains dissolved in the reaction mixture even at a higher temperature and therefore there is no need to heat the reaction mixture. At the temperature in the range of from 5 to 10 °C, the alkaline metal salt of 5,5´-azotetrazole precipitates as a solid. The product is isolated by filtration.

In one embodiment, the above-prepared alkaline metal salt of 5,5´-azotetrazole is subsequently subjected to cationic exchange *in situ*. The cation exchange is preferably carried out by reaction with an aqueous solution of an alkaline earth metal salt, an aqueous solution of ammonia or an aqueous solution of an organic nitrogenous base containing the group 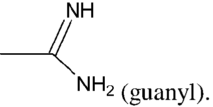 Alkaline earth metals are Mg, Ca, Sr and Ba.

The alkaline earth metal salt is a salt formed by the reaction of an alkaline earth metal hydroxide (group II.A of the periodic table) with an inorganic acid, preferably selected from the group comprising HCl, HNO₃, H₂CO₃, H₃PO₄, H₂SO₄, or with an organic acid, preferably selected from the group comprising acetic acid, formic acid. Preferably, alkaline earth salts are selected from the group comprising chlorides, nitrates, carbonates, bicarbonates, sulphates, hydrogen sulphates, acetates, formates, phosphates.

The organic nitrogenous base containing the guanyl group may be in the form of a chloride, sulfate, nitrate, carbonate or a free base, e.g. guanidine. In a preferred embodiment, the organic nitrogenous base containing guanyl is selected from the group comprising guanidium chloride, triaminoguanidium chloride, guanylurea chloride.

Examples of suitable cationic exchange reactants are BaCl₂, guanidium hydrochloride, triaminoguanidium hydrochloride or guanylurea hydrochloride. In this way, it is possible to prepare *in situ* for example a salt of 5,5´-azotetrazole with Ba²⁺ ions, with guanidinium, triaminoguanidinium or with guanylurea.

Cationic exchange occurs at any temperature (in solution or in suspension). The reaction proceeds better if the reactants are in solution, i.e. at elevated temperature or with sufficient dilution. In one embodiment, said cationic exchange may occur for example at the temperature in the range of from 60 to 80 °C, preferably at the temperature of 70 °C.

After cooling the reaction mixture, comprising the prepared 5,5´-azotetrazole salt, to the temperature in the range of from 5 to 10 °C, said salt may be precipitated/crystallized, which simplifies its subsequent isolation, for example by filtration, centrifugation, sedimentation, etc.

The advantage of the present invention is the improved and simple method of synthesis of alkaline metal salts of 5,5´-azotetrazole, which reduces production costs by using cheap and available oxidizing agents with significantly lower toxicity compared to chlorine or bromine (SDIC and TCCA are used in swimming pool chemistry). At the same time, the problematic filtration of manganese dioxide and the need to evaporate large volumes of water or dilute the reaction mixture with ethanol are eliminated. Eliminating water evaporation is advantageous both because of the reduction in energy requirements and because of the reduction in the risk of the formation of more sensitive and dangerous lower hydrates or anhydrous salts (due to the potential loss of crystal waters), the sensitivity of which is at the level of explosives.

Another advantage of the solution according to the invention is the possibility of direct preparation of other salts of 5,5'-azotetrazole from its alkali metal salts, formed in the reaction mixture, *in situ*. This solution includes the preparation of other inorganic and organic salts such as ammonium and barium salts of 5,5'-azotetrazole, nitrogenous substances containing an amidino group in its molecule (CNHNH₂, formerly guanyl), for example guanidinium azotetrazolate, diaminoguanidinium azotetrazolate, triaminoguanidinium azotetrazolate, guanylurea azotetrazolate, using the reaction of the alkali metal salt of 5,5'-azotetrazole with organic nitrogenous base or with the cation thereof or with the relevant metal cation.

### Examples

### Example 1: Synthesis of sodium azotetrazolate using sodium salt of dichloroisocyanuric acid

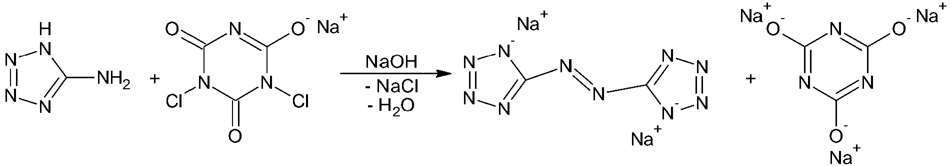

8.5 g (0.1 mol) of anhydrous 5-aminotetrazole and 30 g (0.75 mol) of sodium hydroxide were dissolved in 140 ml of water in a 400ml beaker while stirred using a mechanical stirrer, and the solution was then cooled to the temperature of 0-3 °C using a water bath with sodium chloride. 13 g (0.059 mol) of sodium salt of dichloroisocyanuric acid (SDIC) were dissolved in 70 ml of water at room temperature, and this solution was then added dropwise from the separatory funnel into the reaction mixture of 5-aminotetrazole in such a way, so that the temperature of the reaction mixture would remain in the range of 0-3 °C. Yellowing of the reaction mixture was immediate and foaming was minimal. Dosing SDIC solution took place during cca 30 minutes, and was followed by stirring for further 60 minutes at 0 °C. Then heating was started and when the temperature reached 87-90 °C, hot filtration of trisodium salt of cyanuric acid was performed. After filtration, the filter cake was washed with 5 ml of hot water. The filtrate was then cooled to 5-10 °C, whereupon solid sodium salt of 5,5´-azotetrazole precipitated, and filtration and washing of the product with water were performed. The reaction product was sodium 5,5'-azotetrazolate pentahydrate in the form of easily filterable crystals. The yield of the reaction was 12.7 g (85 %). M.p. 273-276 °C. DTA: *1. endotherm*: 55 °C (*start*), 106 °C (*minimum*); *2. endotherm*: 122 °C (*start*), 146 °C (*minimum*); *exotherm*: 212 °C (*start*), 239 °C (*maximum*). Elemental analysis, *calculated for* C₂H₁₀N₁₀Na₂O₅ (%): C 8.00, H 3.36, N 46.67. *Found:* C 8.08, H 3.26, N 46.78. FTIR (cm⁻¹): 3584, 3538, 3368, 3243, 3100, 2833, 2447, 2107, 1684, 1625, 1480, 1452, 1400, 1204, 1165, 1097, 1065, 1043, 926, 774, 739 a 730, 559, 508, 401.

### Example 2: Synthesis of sodium azotetrazolate using N-bromosuccinimide

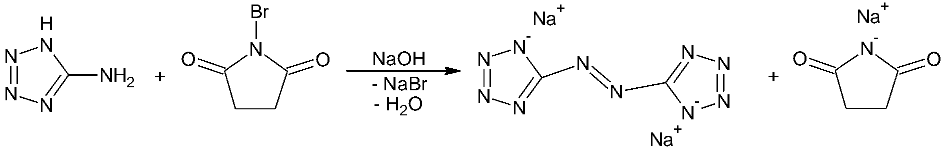

8.5 g (0.1 mol) of anhydrous 5-aminotetrazole and 30 g (0.75 mol) of sodium hydroxide were dissolved in 150 ml of water in a 250ml beaker while stirred using an electromagnetic stirrer. The solution was then cooled down to the temperature of 0 °C using an ice bath with sodium chloride. 18 g (0.1 mol) of *N*-bromosuccinimide was then added in small portions to the reaction mixture, while the temperature was kept at 0-3 °C. Yellowing of the reaction mixture was immediate and foaming was minimal. Dosing of *N*-bromosuccinimide took place during 30 minutes, and was followed by stirring for further 60 minutes at 0 °C. At this temperature, the reaction by-product stayed dissolved in the reaction mixture, while the solid pentahydrate of sodium 5,5´-azotetrazolate precipitated and was isolated by filtration and washed with water. The yield of the reaction was 13.1 g (88 %). M.p. 273-276 °C. DTA: *1. endotherm* 56 °C *(start)*, 108 °C *(minimum); 2*. *endotherm*: 123 °C (start), 153 °C (*minimum*); *exotherm*: 214 °C (*start*), 240 °C (*maximum*). Elemental analysis, *calculated for* C₂H₁₀N₁₀Na₂O₅ (%): C 8.00, H 3.36, N 46.67. *Found:* C 8.15, H 3.31, N 46.35. FTIR (cm⁻¹): 3584, 3538, 3368, 3243, 3100, 2833, 2447, 2107, 1684, 1625, 1480, 1452, 1400, 1204, 1165, 1097, 1065, 1043, 926, 774, 739, 730, 559, 508, 401.

### Example 3: Synthesis of sodium azotetrazolate using N-bromosuccinimide

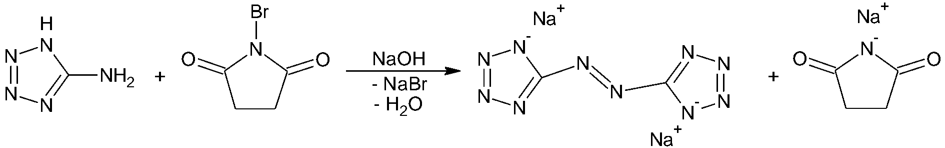

8.5 g (0.1 mol) of anhydrous 5-aminotetrazole and 30 g (0.75 mol) of sodium hydroxide were dissolved in 150 ml of water in a 250ml beaker while stirred using an electromagnetic stirrer. The solution was then cooled down to the temperature of 10 °C using tap water. 18 g (0.1 mol) of *N-*bromosuccinimide was then added in small portions to the reaction mixture, while the temperature was kept at 10-15 °C. . Yellowing of the reaction mixture was immediate and foaming was minimal. Dosing of *N*-bromosuccinimide took place during 30 minutes, and was followed by stirring for further 60 minutes at 10 °C. The reaction by-product stayed dissolved in the reaction mixture, while the solid pentahydrate of sodium 5,5´-azotetrazolate precipitated and was isolated by filtration. The product was washed with water. The reaction yield was 12.8 g (85 %). M.p. 273-276 °C. DTA: *1. endotherm* 57 °C *(start)*, 107 °C *(minimum); 2*. *endotherm*: 125 °C (start), 152 °C (*minimum*); *exotherm*: 216 °C (*start*), 242 °C (*maximum*). Elemental analysis, *calculated for* C₂H₁₀N₁₀Na₂O₅ (%): C 8.00, H 3.36, N 46.67. *Found:* C 8.12, H 3.25, N 46.54. FTIR (cm⁻¹): 3584, 3538, 3368, 3243, 3100, 2833, 2447, 2107, 1684, 1625, 1480, 1452, 1400, 1204, 1165, 1097, 1065, 1043, 926, 774, 739, 730, 559, 508, 401.

### Example 4: Synthesis of potassium azotetrazolate using trichloroisocyanuric acid

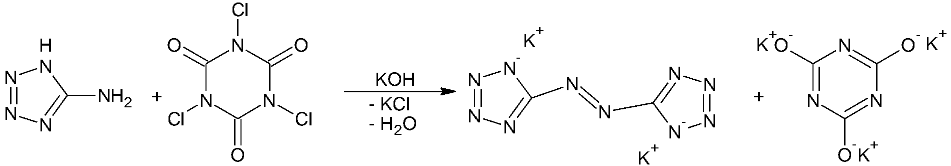

The reaction mixture contained 8.5 g (0.1 mol) of 5-aminotetrazole, 45 g (0.8 mol) of potassium hydroxide and 170 ml of water. 9 g (0.039 mol) of trichloroisocyanuric acid was added to this solution under the same conditions as in Example 1. The procedure was then the same as in Example 1. The reaction product was isolated from the reaction mixture by precipitation using 170 ml of methanol. The reaction yield was 9.7 g (59 %) of pentahydrate of potassium 5,5'-azotetrazolate. DTA: *endotherm* 88 °C *(start)*, 107 °C *(minimum); exotherm*: 220 °C (*start*), 247 °C (*maximum*). Elemental analysis, *calculated for* C₂H₁₀N₁₀K₂O₅ (%): C 7.23, H 3.03, N 42.14. *Found:* C 7.43, H 2.98, N 41.64. FTIR (cm⁻¹): 3550, 3362, 3265, 1650, 1613, 1504, 1479, 1441, 1389, 1199, 1188, 1160, 1154, 1077, 1062, 1052, 1039, 1034, 774, 727, 561, 451, 442, 421, 413, 405.

### Example 5: Synthesis of cesium azotetrazolate using trichloroisocyanuric acid

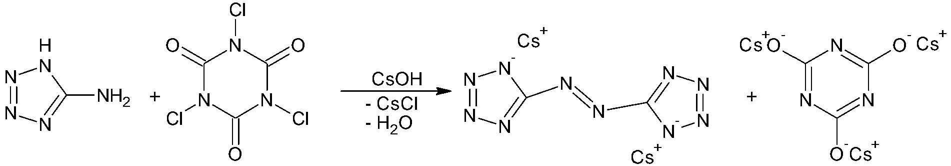

The reaction mixture contained 0.42 g (5 mmol) of 5-aminotetrazole, 7 ml 50 wt.% of cesium hydroxide and 1.5 ml of water. 0.45 g (1.66 mmol) of trichloroisocyanuric acid was added to this solution under the same conditions as in Example 1. The procedure was then the same as in Example 1. The reaction yield was 0.71 g (62 %) of dihydrate of cesium 5,5´-azotetrazolate. DTA: *1. endotherm* 59 °C *(start)*, 63 °C *(minimum); 2.* endotherm: 73 °C (*start*), 87 °C (*minimum*); *exotherm*: 212 °C (*start*), 243.5 °C (*maximum*). Elemental analysis, *calculated for* C₂H₇N₁₀Cs₂O₂ (%): C 5.16, H 0.87, N 30.06. *Found:* C 5.48, H 1.01, N 28.97. FTIR (cm⁻¹): 3243, 1681, 1388, 1366, 1196, 1180, 1153, 1071, 1053, 1040, 1032, 781, 773, 729, 655, 577, 565, 442.

### Example 6: Synthesis of baryum azotetrazolate in situ using trisodium salt of cyanuric acid

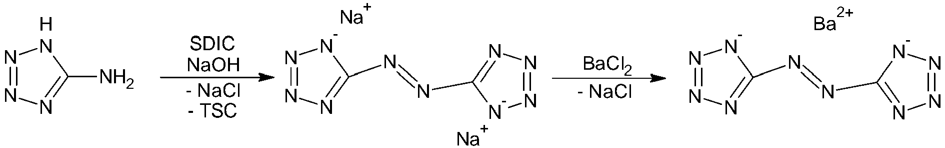

First, the sodium salt of 5,5'-azotetrazole was prepared according to the procedure of Example 1. After filtering off the trisodium salt of cyanuric acid, the filtrate was neutralized with a weak acid at a temperature of 70° C. Any weak acid, for example formic acid, citric acid, acetic acid or also diluted HCl, is suitable for neutralization in this and the following examples. Subsequently, baryum chloride (20.8 g, 0.1 mol) was slowly added to this reaction mixture at the same temperature. After the addition, the reaction mixture was stirred for 1 hour and after cooling the reaction mixture to the temperature of 5-10 °C, the pentahydrate of baryum 5,5´-azotetrazolate was isolated and washed with 2 x 10 ml of water. The reaction yield was 16.7 g (85 %). DTA: *1. endotherm* 101 °C *(start)*, 120 °C *(minimum); 2. endotherm*: 173 °C (*start*), 194 °C (*maximum*); *exotherm*: 208 °C (*start*), 235 °C (*maximum*). Elemental analysis, *calculated for* C₂H₁₀N₁₀ BaO₅ (%): C 6.14, H 2.57, N 35.78. *Found:* C 6.11, H 2.57, N 35.08. FTIR (cm⁻¹): 3585, 3396, 3131, 1621, 1455, 1401, 1200, 1182, 1161, 1096, 1062, 1041, 794, 774, 734, 730, 559, 401.

### Example 7: Synthesis of guanidium azotetrazolate in situ using trisodium salt of cyanuric acid

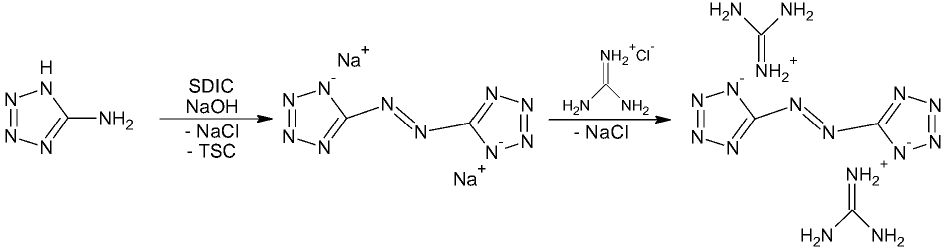

First, the sodium salt of 5,5'-azotetrazole was prepared according to the procedure of Example 1. After filtering off the trisodium salt of cyanuric acid, the filtrate was neutralized with a weak acid at a temperature of 70° C. Subsequently, 15 ml (19.1 g, 0.2 mol) of an aqueous solution of guanidium hydrochloride was slowly added dropwise to this reaction mixture at the same temperature. After the addition, the reaction mixture was stirred for 2 hours at the same temperature, and after cooling the reaction mixture to the temperature of 5-10 °C, the solid guanidium 5,5'-azotetrazolate was isolated and washed with 2 x 10 ml of water and 10 ml of ethanol. The reaction yield was 10.2 g (72 %). DTA: *exotherm*: 231 °C (*start*), 243 °C (*maximum*). Elemental analysis, *calculated for* C₂H₁₀N₁₀ (%): C 16.90, H 4.26, N 78.84. *Found:* C 16.83, H 4.20, N 78.12. FTIR (cm⁻¹): 3438, 3388, 3316, 3187, 3080, 2825, 1695, 1648, 1584, 1568, 1454, 1398, 1196, 1174, 1160, 1081, 1048, 1010, 768, 735, 691, 569, 531, 489, 434.

### Example 8: Synthesis of triaminoguanidium azotetrazolate in situ using trisodium salt of cyanuric acid

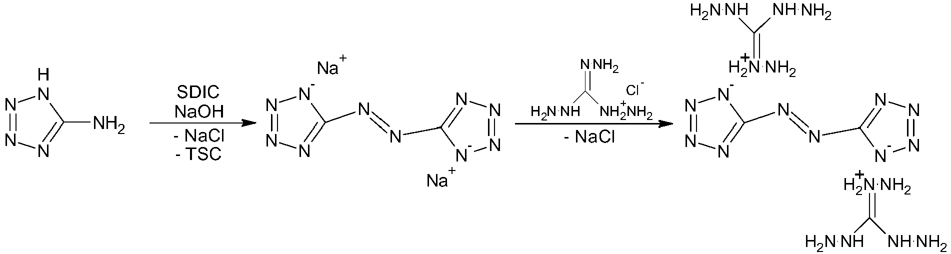

First, the sodium salt of 5,5'-azotetrazole was prepared according to the procedure of Example 1. After filtering off the trisodium salt of cyanuric acid, the filtrate was neutralized with a weak acid at a temperature of 70° C. Subsequently, triaminoguanidium-hydrochloride (4.64 g, 0.033 mol) was slowly added to the reaction mixture at the same temperature. After the addition, the reaction mixture was stirred for 1 hour and after cooling the reaction mixture to the temperature of 5-10 °C, solid triaminoguanidium 5,5´-azotetrazolate was isolated, and washed with 2 x 10 ml of water and 10 ml of ethanol. The reaction yield was 4.67 g (75 %). DTA: *exotherm*: 174.5 °C (*start*), 195 °C (*maximum*). Elemental analysis, *calculated for* C₄H₁₈N₂₂ (%): C 12.83, H 4.85, N 82.32. *Found:* C 12.84, H 4.88, N 81.13. FTIR 3351, 3333, 3199, 2799, 1674, 1630, 1596, 1567, 1435, 1385, 1332, 1186, 1160, 1135, 1068, 1033, 990, 941, 770, 730, 636, 584, 559, 444; 418.

### Example 9: Synthesis of guanylurea azotetrazolate in situ using trisodium salt of cyanuric acid

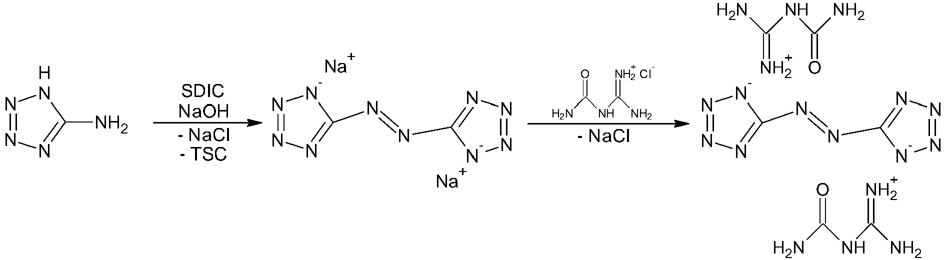

First, the sodium salt of 5,5'-azotetrazole was prepared according to the procedure of Example 1. After filtering off the trisodium salt of cyanuric acid, the filtrate was neutralized with a weak acid at a temperature of 70° C.

Subsequently, 20 ml (13.86 g, 0.1 mol) of an aqueous solution of guanylurea hydrochloride was slowly added dropwise to this reaction mixture at the same temperature. After the addition, the reaction mixture was stirred for 2 hours, when the product is formed and filtered off at 70 °C. Solid hemihydrate of guanylurea 5,5'-azotetrazolate was isolated and washed with 2 x 10 ml of ice-cold water and 10 ml of ethanol. The reaction yield was 14.27 g (75 %). DTA: *exotherm*: 205 °C (*start*), 218,5 °C (*maximum*). Elemental analysis, *calculated for* C₆H₁₅N₁₈O_{2,5} (%): C 19.00, H 3.99, N 66.46. *Found:* C 19.37, H 3.77, N 66.53. FTIR (cm⁻¹): 3448, 3394, 3313, 3194, 2790, 1717, 1685, 1639, 1586, 1528, 1465, 1446, 1396, 1353, 1194, 1129, 1088, 1079, 1040, 1027, 930, 831, 768; 737, 701, 570, 524, 486, 445, 428.

### Example 10: Synthesis of guanidium azotetrazolate in situ using N-bromosuccinimide

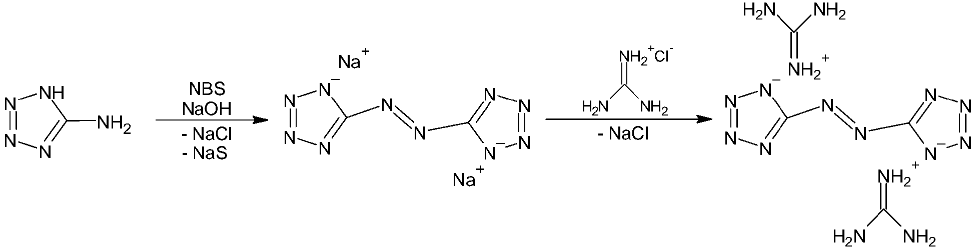

First, the sodium salt of 5,5'-azotetrazole was prepared according to the procedure of Example 2. Sodium azotetrazolate was not isolated from the solution and the reaction mixture was neutralized with a weak acid at a temperature of 70° C. Subsequently, guanidium hydrochloride 19.1 g (0.2 mol) was slowly added to this reaction mixture at the same temperature. After the addition, the reaction mixture was stirred for 1 hour and after cooling the reaction mixture to the temperature of 5-10 °C, guanidinium 5,5´-azotetrazolate was isolated and washed with 2 x 10 ml of water and 10 ml of ethanol. The reaction yield was 10.7 g (75 %). DTA: *exotherm*: 230 °C (*start*), 245 °C (*maximum*). Elemental analysis, *calculated for* C₂H₁₀N₁₀ (%): C 16.90, H 4.26, N 78.84. *Found:* C 16.75, H 4.11, N 77.96. FTIR (cm⁻¹): 3438, 3387, 3317, 3189, 3079, 2825, 1695, 1648, 1584, 1568, 1398, 1196, 1175, 1160, 1081, 1048,1040, 1010, 768, 735, 691, 569, 530, 493, 434.

### Industrial Applicability

The present method simplifies and reduces the cost of producing 5,5'-azotetrazole salts and thus the substances derived from them. The substances prepared in this way are useful in particular as components of gas generators and green propellants (guanidinium azotetrazolate, diaminoguanidinium 5,5'-azotetrazolate, triaminoguanidinium 5,5'-azotetrazolate, guanylurea 5,5'-azotetrazolate, 5,5'-bishydrazotetrazole, ammonium salt of 5,5'-azotetrazolate), explosives (lead and bismuth salt of 5,5'-azotetrazolate), or as powerful explosives/high explosives (5,5'-hydrazobistetrazole, guanylurea 5,5'-azotetrazolate).

## Claims

1. A method of preparation of 5,5'-azotetrazole salts, **characterized in that** it comprises a step of oxidation of 5-aminotetrazole with an oxidizing agent in an aqueous solution of alkali metal hydroxide, wherein said oxidizing agent is selected from the group consisting of trichloroisocyanuric acid, dichloroisocyanuric acid, sodium dichloroisocyanurate, N-chlorosuccinimide, and *N*-bromosuccinimide, to form alkaline metal salt of 5,5'-azotetrazole.

2. The method according to claim 1, **characterized in that** the oxidation is performed at the temperature in the range of from -10 to 80 °C.

3. The method according to claim 1 or 2, **characterized in that** the oxidation is performed for at least 5 min, preferably for at least 60 min.

4. The method according to claim 1, 2 or 3, **characterized in that** the alkali metal hydroxide is selected from the group comprising NaOH, KOH, LiOH, CsOH and RbOH.

5. The method according to any one of the claims 1 to 4, **characterized in that** the concentration of the alkali metal hydroxide in the reaction mixture is in the range of from 0.1 to 12 mol/dm³.

6. The method according to any one of the claims 1 to 5, **characterized in that** the initial concentration of 5-aminotetrazole in the reaction mixture is in the range of from 0.1 to 3 mol/dm³.

7. The method according to any one of the claims 1 to 6, **characterized in that** the oxidation step of 5-aminotetrazole is followed by a step of removal of solid by-products of the reaction, wherein the reaction mixture is heated to the temperature in the range of from 35 to 95 °C, and filtered at a temperature in said range; wherein the filtrate contains the prepared alkaline metal salt of 5,5'-azotetrazole.

8. The method according to any one of the claims 1 to 7, **characterized in that** the alkaline metal salt of 5,5'-azotetrazole, prepared according to any one of the preceding claims 1 to 7, is subsequently subjected to cationic exchange *in situ.*

9. The method according to claim 8, **characterized in that** the cationic exchange is performed at the temperature in the range of from 60 to 80 °C, preferably at the temperature of 70 °C.

10. The method according to any one of the preceding claims, **characterized in that** it is followed by cooling down the reaction mixture comprising the prepared 5,5´-azotetrazole salt to the temperature in the range of from 5 to 10 °C to form solid 5,5´-azotetrazole salt, which is subsequently isolated.
